# EUROPÄISCHE PATENTSCHRIFT

(11) **EP 0 639 561 B1**
(45) Veröffentlichungstag und Bekanntmachung des Hinweises auf die Patenterteilung: **02.04.1997**
(21) Anmeldenummer: 94112193.1
(22) Anmeldetag: 04.08.1994
(51) Int. Cl.: C07C 317/14, C07C 315/04

(54) **Verfahren zur Herstellung von Alkyl-(3-chlorphenyl)-sulfonen**
Process for the production of Alkyl-(3-chlorophenyl) sulfones
Procédé pour la préparation d'alkyl (chloro-3 phényl) sulfones

(30) Priorität: 17.08.1993 DE 4327571
(43) Veröffentlichungstag der Anmeldung: 22.02.1995
(73) Patentinhaber: BAYER AG, 51368 Leverkusen (DE)
(72) Erfinder: Antons, Stefan, Dr., D-51373 Leverkusen (DE); Fiege, Helmut, Dr., D-51373 Leverkusen (DE); Bussmann, Werner, Dr., D-51373 Leverkusen (DE); Richter, Hartmut, Dr., D-40764 Langenfeld (DE)

(56) Entgegenhaltungen:
- WO-A-91/07384
- WO-A-92/14700
- DD-A- 260 493
- DE-C- 949 054
- PATENT ABSTRACTS OF JAPAN, unexamined applications, C Field, Band 14, Nr. 435, 18. September 1990 THE PATENT OFFICE JAPANESE GOVERNMENT Seite 69 C 760; & JP-A-02 169 564 (NIPPON KAYAKU)
- PATENT ABSTRACTS OF JAPAN, unexamined applications, C Field, Band 15, Nr. 392, 4. Oktober 1991 THE PATENT OFFICE JAPANESE GOVERNMENT Seite 132 C 873; & JP-A-03 161 469 (NISSAN CHEM IND)
- CHEMICAL ABSTRACTS, Band 62, Nr. 6, 15. MUrz 1965, Columbus, Ohio, USA HIROSHI KUGITA et al. "Duiretics I. Alkylsulfonyl- toluene derivatives" Spalte 6422,

## Beschreibung

Die vorliegende Erfindung betrifft ein verbessertes Verfahren zur Herstellung von Alkyl-(3-chlorphenyl)-sulfonen durch Chlorierung von Alkylphenylsulfonen.

Es ist bekannt, daß man Methyl-(4-methylphenyl)-sulfon durch Umsetzung mit Chlor in Gegenwart von Antimon(III)-chlorid ohne Lösungsmittel bei 85 bis 90°C in Methyl-(3-chlor-4-methyl)-phenylsulfon überführen kann (siehe CA 62, 6422 c (1965)). Das nach diesem Verfahren gewonnene Produkt ist jedoch durch höherchlorierte Nebenprodukte verunreinigt (siehe US-A 4 675 447, Seite 1, Zeile 25).

Die Chlorierung von Methyl-(4-methylphenyl)-sulfon mit Sulfurylchlorid in Gegenwart von Metallchloriden ist bei 90 bis 92°C durchgeführt worden (siehe US-A 4 675 447). Die dort beschriebenen Verfahrensergebnisse konnten beim Nacharbeiten nicht bestätigt werden (siehe WO 91/07384). Außerdem werden bei diesem Verfahren große Mengen an Katalysatoren benötigt und die erreichbaren Ausbeuten liegen unter 80 % der Theorie.

Gemäß der DE-PS 949 054 werden 4-Alkylphenylsulfone in Chlorschwefelsäure oder Schwefelsäure in Anwesenheit von Kupfer(I)-halogeniden und Iod chloriert. Hierbei entstehen bevorzugt höherchlorierte Verbindungen (siehe auch Houben-Weyl, Methoden der organischen Chemie, 4. Auflage, Band V/3, S. 715-716 (1962)).

Gemäß der WO 91/07384 werden 3-Chlor-Verbindungen durch Chlorierung in Schwefelsäure oder Oleum hergestellt. Neben der anfallenden Dünnsäure, die Probleme bei der Entsorgung verursacht, entstehen hier offensichtlich wasserlösliche Nebenprodukte, die das Abwasser belasten (siehe Beispiele 1 und 2).

Es besteht somit noch ein Bedürfnis nach einem verbesserten Verfahren zur Herstellung von Alkyl-(3-chlorphenyl)-sulfonen, bei dem die Nachteile der bekannten Verfahren vermieden werden.

Es wurde nun ein Verfahren zur Herstellung von Alkyl-(3-chlorphenyl)-sulfonen durch Chlorierung von Alkylphenylsulfonen gefunden, das dadurch gekennzeichnet ist, daß man Alkylphenylsulfone in geschmolzener Form und in Gegenwart von Eisen(III)-chlorid mit elementarem Chlor chloriert. Gegebenenfalls kann man zusätzlich in Gegenwart von Jod und/oder Schwefel arbeiten.

Das erfindungsgemäße Verfahren eignet sich insbesondere zur Herstellung von Alkyl-(3-chlorphenyl)-sulfonen der Formel (I) 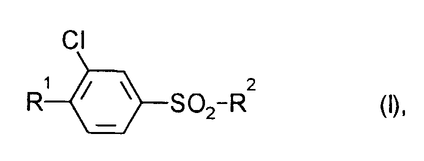 in der
- R¹ und R²: gleich oder verschieden sind und für eine geradkettige oder verzweigte C₁- bis C₄-Alkylgruppe stehen,
aus Alkylphenylsulfonen der Formel (II) 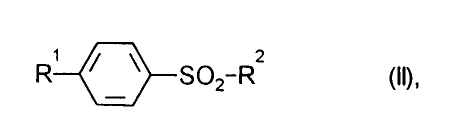 in der
- R¹ und R²: die bei Formel (I) angegebene Bedeutung haben.

Besonders bevorzugt stellt man erfindungsgemäß p-Methyl-(3-chlorphenyl)-methylsulfon aus p-Methylphenyl-methylsulfon her.

Für die vorliegende Erfindung ist es wesentlich, daß man Alkylphenylsulfone in geschmolzener Form, d.h. ohne Zusatz von Säuren oder Lösungsmitteln, in Anwesenheit von Eisen(III)-chlorid und gegebenenfalls Iod und/oder Schwefel mit elementarem Chlor chloriert.

Das erfindungsgemäße Verfahren kann man beispielsweise bei 80 bis 180°C durchführen. Vorzugsweise arbeitet man bei 80 bis 120°C, besonders bevorzugt bei 80 bis 95°C.

Vorzugsweise arbeitet man bei Normaldruck. Man kann gegebenenfalls auch unter Druck arbeiten, beispielsweise bei einem Druck bis zu 10 bar.

Das Einleiten von Chlor führt man beispielsweise mindestens so lange durch, bis wenigstens 90 Gew.-% des eingesetzten Alkylphenylsulfons umgesetzt sind, was man z.B. mittels Gaschromatographie feststellen kann. Vorzugsweise leitet man so lange Chlor ein, bis zwischen 95 und 100 Gew.-% des eingesetzten Alkylphenylsulfons umgesetzt sind. Im allgemeinen werden dazu zwischen 4 und 16 Stunden benötigt.

Eisen(III)-chlorid alleine oder Eisen(III)-chlorid plus Jod und/oder Schwefel können jeweils beispielsweise in Mengen von 0,1 bis 5 Gew.-%, vorzugsweise 0,2 bis 2 Gew.-%, bezogen auf eingesetztes Alkylphenylsulfon, eingesetzt werden. Vorzugsweise liegt diese Menge bei 0,2 bis 1 Gew.-%. Das Gewichtsverhältnis von Eisen(III)-chlorid zu Iod und/oder Schwefel kann beispielsweise 0,1:1 bis 10:1 betragen. Vorzugsweise beträgt es 1:1 bis 5:1.

Nach Durchführung des erfindungsgemäßen Verfahren ist es vorteilhaft, restliches Chlor aus dem Reaktionsgefäß auszublasen, z.B. mit Luft oder Stickstoff.

Das hergestellte Alkyl-(3-chlorphenyl)-sulfon enthält im allgemeinen nur geringe Mengen unumgesetztes Ausgangsprodukt und/oder nur geringe Mengen höher chlorierter Verbindungen. Es kann häufig so, wie es beim erfindungsgemäßen Verfahren anfällt, weiterverarbeitet werden. Gegebenenfalls kann man das hergestellte Alkyl-(3-chlorphenyl)-sulfon reinigen, beispielsweise durch Waschen mit Wasser, Destillieren, Fraktionieren und/oder Umkristallisieren.

Auf die erfindungsgemäße Weise erhält man Alkyl-(3-chlorphenyl)-sulfone im allgemeinen in Ausbeuten von über 90 % der Theorie.

Alkyl-(3-chlorphenyl)-sulfone sind wichtige Zwischenprodukte zur Herstellung von Pflanzenschutzmitteln (siehe beispielsweise EP-A 268 795).

### Beispiele

### Beispiel 1

Zu 190 g geschmolzenem p-Methylphenyl-methylsulfon wurden 3,6 g Eisen(III)-chlorid und 0,71 g Iod hinzugefügt und bei 80 bis 90°C solange Chlor eingeleitet bis eine 95 %ige Umsetzung erreicht war (gaschromatographische Analyse). Es wurden 226 g Methyl-(3-chlorphenyl)-methylsulfon mit einem Gehalt von 93 Gew.-% erhalten. Dieses war für die Weiterverarbeitung geeignet.

### Beispiel 2

Es wurde verfahren wie in Beispiel 1, jedoch wurde bis zu 100 %igem Umsatz chloriert. Es wurde ein 95,5 % reines Produkt erhalten.

### Beispiel 3

Zu 190 g geschmolzenem p-Methylphenyl-methylsulfon wurden 3,63 g Eisen(III)-chlorid und 0,71 g Iod hinzugefügt und bei 90°C im Verlaufe von 11 Stunden 115 g Chlor eingeleitet. Danach betrug der Umsatz 95,2 %. Es wurden 227,5 g Methyl-(3-chlorphenyl)-methylsulfon mit einem Gehalt von 92,7 % erhalten (= 92 % der Theorie).

### Beispiel 4

Zu 80 g geschmolzenem p-Methylphenyl-methylsulfon wurden 1,6 g Eisen(III)-chlorid und 0,31 g Iod hinzugefügt und bei 90°C solange Chlor eingeleitet, bis der Umsatz 99,8 %ig war. Es wurden 102,7 g Methyl-(3-chlorphenyl)-methylsulfon mit einer Reinheit von 91,8 % erhalten, was einer Ausbeute von 92,2 % der Theorie entspricht.

### Beispiel 5 (zum Vergleich mit der DE-PS 949 054)

100 g p-Methylphenyl-methylsulfon wurden mit 20 g 96 gew.-%iger Schwefelsäure versetzt und 5 Mol-% Kupfer(I)-chlorid und 5 Mol-% Iod hinzugefügt und dann bei 30°C über 100 Mol-% Chlor eingeleitet. Danach betrug der Umsatz 27,5 % und die Selektivität der Bildung von Methyl-(3-chlorphenyl)-methylsulfon 95,9 %.

### Beispiel 6

Zu 170 g p-Methylphenyl-methylsulfon wurden unter Ausschluß von Luftfeuchtigkeit in einer Chlorierapparatur 1,6 g Eisen(III)-chorid und 1,6 g Schwefel hinzugefügt und bei 80 bis 90°C Chlor eingeleitet. Nachdem ein Umsatz von 99 % erreicht war wurde die Chlorzufuhr abgestellt und aus dem Reaktionsgemisch restliches Chlor mit Stickstoff ausgeblasen. Nach dem Waschen des Rohproduktes mit verdünnter Salzsäure und Wasser und anschließendem Trocknen wurde über 97 % reines Methyl-(3-chlorphenyl)-methylsulfon in einer Ausbeute von 96 % der Theorie erhalten.

### Beispiel 7

Es wurde verfahren wie in Beispiel 6, jedoch wurden nur 0,64 g Schwefel eingesetzt. Es wurde ein 97 % reines Methyl-(3-chlorphenyl)-methylsulfon in einer Ausbeute von 95,5 % der Theorie erhalten.

### Beispiel 8

Es wurde verfahren wie in Beispiel 6, jedoch wurden 3,24 g Eisen(III)-chlorid und 1,24 g Schwefel eingesetzt. Es wurde ein 96,5 % reines Produkt bei einem Umsatz von 99 % erhalten.

### Beispiel 9 (zum Vergleich mit WO 91/07384)

85 g p-Methylphenyl-methylsulfon wurden in 160 g 96 %iger Schwefelsäure gelöst und bei 80°C chloriert. Nach einem Umsatz von 99,5 % wurde nach Aufarbeitung und Neutralwäsche ein 93,8 % reines Methyl-(3-chlorphenyl)-methylsulfon in einer Ausbeute von 89 % der Theorie erhalten.

### Beispiel 10

Es wurde verfahren wie in Beispiel 6, jedoch ohne die Zugabe von Schwefel. Nach einem Umsatz von 98 % wurde ein 92 % reines Produkt erhalten.

## Patentansprüche

1. Verfahren zur Herstellung von Alkyl-(3-chlorphenyl)-sulfonen durch Chlorierung von Alkylphenylsulfonen, dadurch gekennzeichnet, daß man Alkylphenylsulfone in geschmolzener Form und in Gegenwart von Eisen(III)-chlorid mit elementarem Chlor chloriert.

2. Verfahren nach Anspruch 1, dadurch gekennzeichnet, daß man Alkyl-(3-chlorphenyl)-sulfone der Formel (I) 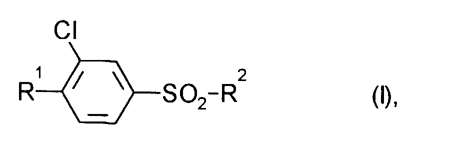 in der
R¹ und R² gleich oder verschieden sind und für eine geradkettige oder verzweigte C₁- bis C₄-Alkylgruppe stehen,
aus Alkylphenylsulfonen der Formel (II) 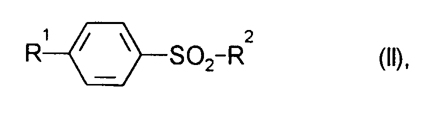 in der
R¹ und R² die bei Formel (I) angegebene Bedeutung haben,
herstellt.

3. Verfahren nach Ansprüchen 1 und 2, dadurch gekennzeichnet, daß man p-Methyl-(3-chlorphenyl)-methylsulfon aus p-Methylphenyl-methylsulfon herstellt.

4. Verfahren nach Ansprüchen 1 bis 3, dadurch gekennzeichnet, daß man es bei 80 bis 180°C durchführt.

5. Verfahren nach Ansprüchen 1 bis 4, dadurch gekennzeichnet, daß man solange Chlor einleitet, bis wenigstens 90 Gew.-% des eingesetzten Alkylphenylsulfons umgesetzt sind.

6. Verfahren nach Ansprüchen 1 bis 5, dadurch gekennzeichnet, daß man zusätzlich in Gegenwart von Jod und/oder Schwefel arbeitet.

7. Verfahren nach Anspruch 6, dadurch gekennzeichnet, daß Eisen(III)-chlorid alleine oder Eisen(III)-chlorid plus Iod und/oder Schwefel jeweils in Mengen von 0,1 bis 5 Gew.-%, bezogen auf eingesetztes Alkylphenylsulfon, eingesetzt werden.

8. Verfahren nach Ansprüchen 6 und 7, dadurch gekennzeichnet, daß das Gewichtsverhältnis von Eisen(III)-chlorid zu Iod und/oder Schwefel 0,1:1 bis 10:1 beträgt.

9. Verfahren nach Ansprüchen 1 bis 8, dadurch gekennzeichnet, daß man nach der Durchführung des erfindungsgemäßen Verfahrens restliches Chlor aus dem Reaktionsgefäß ausbläst.

10. Verfahren nach Ansprüchen 1 bis 9, dadurch gekennzeichnet, daß man das erhaltene Produkt durch Waschen mit Wasser, Destillieren, Fraktionieren und/oder Umkristallisieren reinigt.

## Claims

1. Process for preparing alkyl-(3-chlorophenyl)-sulphones by chlorination of alkylphenylsulphones, characterized in that alkylphenylsulphones are chlorinated in molten form with elemental chlorine in the presence of iron(III) chloride.

2. Process according to Claim 1, characterized in that alkyl-(3-chlorophenyl)-sulphones of the formula (I) 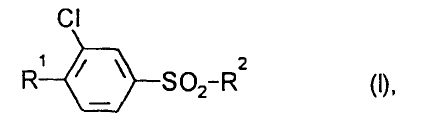 in which
R¹ and R² are identical or different and represent a straight-chain or branched C₁- to C₄-alkyl group,
are prepared from alkylphenylsulphones of the formula (II) 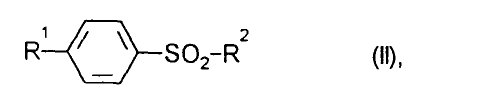 in which
R¹ and R² have the meanings specified for formula (I).

3. Process according to Claims 1 and 2, characterized in that p-methyl-(3-chlorophenyl)-methylsulphone is prepared from p-methylphenyl-methylsulphone.

4. Process according to Claims 1 to 3, characterized in that it is carried out at from 80 to 180°C.

5. Process according to Claims 1 to 4, characterized in that chlorine is passed in until at least 90% by weight of the alkylphenylsulphone used have been converted.

6. Process according to Claims 1 to 5, characterized in that it is carried out in the additional presence of iodine and/or sulphur.

7. Process according to Claim 6, characterized in that iron(III) chloride alone or iron(III) chloride plus iodine and/or sulphur are each used in amounts of from 0.1 to 5% by weight, based on alkylphenylsulphone used.

8. Process according to Claims 6 and 7, characterized in that the weight ratio of iron(III) chloride to iodine and/or sulphur is from 0.1:1 to 10:1.

9. Process according to Claims 1 to 8, characterized in that after carrying out the process of the invention residual chlorine is blown out of the reaction vessel.

10. Process according to Claims 1 to 9, characterized in that the product obtained is purified by washing with water, distillation, fractionation and/or recrystallization.

## Revendications

1. Procédé de préparation d'alkyl-(3-chlorophényl)-sulfones par chloruration d'alkylphénylsulfones, caractérisé en ce que l'on chlore les alkyl-phénylsulfones à l'état fondu et en présence de chlorure de fer-III à l'aide du chlore élémentaire.

2. Procédé selon la revendication 1, caractérisé en ce que l'on prépare des alkyl-(3-chlorophényl)-sulfones de formule (I): 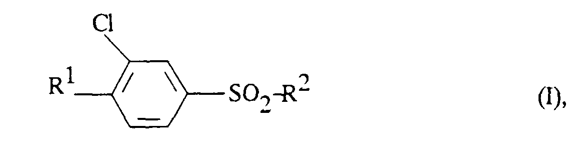 dans laquelle
R¹ et R², ayant des significations identiques ou différentes, représentent chacun un groupe alkyle à chaîne droite ou ramifiée en C₁-C₄,
à partir d'alkylphénylsulfones de formule (II) : 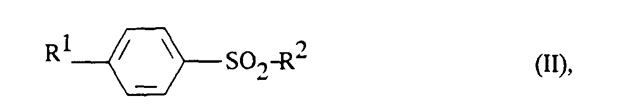 dans laquelle
R¹ et R² ont les significations indiquées en référence à la formule (I).

3. Procédé selon les revendications 1 et 2, caractérisé en ce que l'on prépare la p-méthyl-(3-chlorophényl)-méthylsulfone à partir de la p-méthyl-phényl-méthylsulfone.

4. Procédé selon les revendications 1 à 3, caractérisé en ce que l'on opère à des températures de 80 à 180°C.

5. Procédé selon les revendications 1 à 4, caractérisé en ce que l'on injecte du chlore jusqu'à un taux de conversion d'au moins 90 % en poids de l'alkylphénylsulfone mise en oeuvre;

6. Procédé selon les revendications 1 à 5, caractérisé en ce que l'on opère en outre en présence d'iode et/ou de soufre.

7. Procédé selon la revendication 6, caractérisé en ce que l'on utilise le chlorure de fer-III seul ou avec de l'iode et/ou du soufre, en quantité de 0,1 à 5 % du poids de l'alkylphénylsulfone mise en oeuvre.

8. Procédé selon les revendications 6 et 7, caractérisé en ce que les proportions relatives en poids entre le chlorure de fer-III et l'iode et/ou le soufre sont de 0,1:1 à 10:1.

9. Procédé selon les revendications 1 à 8, caractérisé en ce que, après mise en oeuvre du procédé selon l'invention, on élimine le chlore résiduel du récipient de réaction par soufflage.

10. Procédé selon les revendications 1 à 9, caractérisé en ce que l'on purifie le produit obtenu par lavage à l'eau, distillation, fractionnement et/ou recristallisation.
